**Europäisches Patentamt**

**European Patent Office**

**Office européen des brevets**

(11) Veröffentlichungsnummer : **0 115 548**
**B1**

(12) **EUROPÄISCHE PATENTSCHRIFT**

(45) Veröffentlichungstag der Patentschrift :
01.10.86

(21) Anmeldenummer : 83100989.9

(22) Anmeldetag : 03.02.83

(51) Int. Cl.⁴ : **A 61 B 5/02**, A 61 B 5/00,
**A 61 M 25/00**

(54) In einen Katheter einsetzbarer Messfühler, insbesondere Druckmessfühler.

(43) Veröffentlichungstag der Anmeldung :
15.08.84 Patentblatt 84/33

(45) Bekanntmachung des Hinweises auf die Patenterteilung : 01.10.86 Patentblatt 86/40

(84) Benannte Vertragsstaaten :
CH DE FR GB IT LI NL SE

(56) Entgegenhaltungen :
EP-A- 0 006 577
EP-A- 0 036 171
WO-A-81 /036 13
DE-A- 2 708 607
US-A- 3 490 441
US-A- 3 748 623
US-A- 4 020 830
US-A- 4 274 423
IEEE TRANSACTIONS ON ELECTRON DEVICES,
Band ED-29, Nr. 1, Januar 1982, Seiten 57-63, New
York, USA, M. ESASHI et al.: "Fabrication of cathetertip and sidewall miniature pressure sensors"

(73) Patentinhaber : HONEYWELL MEDICAL ELECTRO-
NICS B.V.
I.B.C.-Weg 1
NL-5683 PK Best (NL)

(72) Erfinder : Beerens, Petrus Hubertus Johannus
Roostenlaan 53
NL-5644 GB Eindhoven (NL)

(74) Vertreter : Rentzsch, Heinz et al
Honeywell Europe S.A. Holding KG Kaiserleistrasse
55 Postfach 184
D-6050 Offenbach am Main (DE)

## Beschreibung

Die Erfindung betrifft einen Meßfühler gemäß Oberbegriff des Anspruchs 1. Ein aus US-A 42 74 423 bekannter Meßfühler dieser Art besteht aus einem im wesentlichen zylindrischen Träger, dessen Mittelstück abgeflacht ist und auf der in Längsrichtung verlaufenden Schnittfläche des Trägers den Halbleiterfühler trägt. Die beiden Endstücke des Trägers haben einen dem Innendurchmesser des Katheters angepaßten Außendurchmesser. Das Mittelstück und das der Katheterspitze abgewandte Endstück des Trägers sind ferner mit einem nutförmigen Kanal versehen, über den ein Referenzdruck auf die Unterseite der als Druckfühler dienenden Halbleitermembran geführt werden kann. Der Meßfühler paßt nur in Katheter vorgegebener Durchmessers.

Ferner ist aus EP-B1 6577 ein Druckmeßkatheter zum Einführen in ein Gefäß eines Patienten bekannt, der über seine Länge verteilt, mindestens zwei Druckwandler zum Messen des Flüssigkeitsdruckes im Gefäß an verschiedenen Stellen aufweist. Jeder Druckwandler ist mit einer teils hohlzylindrischen Meßzelle versehen, die an einem Ende geschlossen ist. Mit ihrem offenen Ende ragt die Meßzelle in eine über eine Öffnung in der Katheterwand mit der Umgebung des Katheters in Verbindung stehenden Kammer des Katheters hinein. Im Innenraum der Meßzelle befinden sich zwei Elektroden zur Messung des elektrischen Widerstands der in die Meßzelle hineingedrückten Flüssigkeitssäule.

Aufgabe der Erfindung ist es, einen Meßfühler, z. B. mit Halbleiter-Druckwandler so auszubilden, daß er auf möglichst einfache Weise in einem Katheter befestigt werden kann, wenig Platz benötigt, gegen mechanische Beanspruchungen geschützt ist und möglichst vielseitig anwendbar ist.

Diese Aufgabe wird gelöst durch die im Anspruch 1 gekennzeichnete Erfindung. Sie ermöglicht die Verwendung eines einheitlichen Meßfühlers in Kathetern unterschiedlichen Durchmessers ; es kann sowohl in der Katheterspitze als auch an anderer Stelle längs des Katheters leicht angebracht werden. Der zwischen der Außenwand des Fühlers und der Innenwand des Katheters frei bleibende Querschnitt ermöglicht eine Zufuhr oder Entnahme von Flüssigkeiten oder die Durchleitung von Anschlußleitungen für weitere Fühler. Vorteilhafte Weiterbildungen ergeben sich aus den Unteransprüchen.

Die Erfindung wird nachfolgend anhand in den Zeichnungen wiedergegebener Ausführungsbeispiele erläutert. Dabei zeigt

Figur 1 einen Längsschnitt durch eine Katheterspitze mit eingesetztem Druckmeßfühler und

Figur 2 im Querschnitt die Lage des Meßfühlers in Kathetern unterschiedlichen Durchmessers.

Auf das Ende eines mit seitlichen Öffnungen 1 versehenen biegsamen Katheters 2 ist eine rohrförmige Stahlkappe 3 aufgesetzt, welche eine seitliche Öffnung 4 aufweist. Hinter der Öffnung sitzt ein Meßfühler 5, der als druckempfindliches Element eine Halbleitermembran, beispielsweise eine mit Dehnelementen versehene Siliziumscheibe aufweist. Der Aufbau solcher Halbleiter-Druckwandler in Miniaturgröße ist bekannt und nicht Gegenstand der vorliegenden Erfindung. Der Meßfühler besteht aus einem Kunststoff- oder Glassubstrat 6, welches als Träger für den eigentlichen Halbleiterfühler 7 dient. Er ist beispielsweise mit Silikongummi 8 in das Stahlrohr 6 eingeklebt und ggf. zusätzlich an den Übergangsstellen 9 mit Epoxydharz abgedichtet. Über einen Rohranschluß 10 steht er mit einem im Katheter 2 geführten Schlauch 11 in Verbindung, über den dem Meßfühler ein Referenzdruck zugeleitet werden kann und der gleichzeitig die elektrischen Anschlußleitungen 12 für den Druckwandler 5 umgibt.

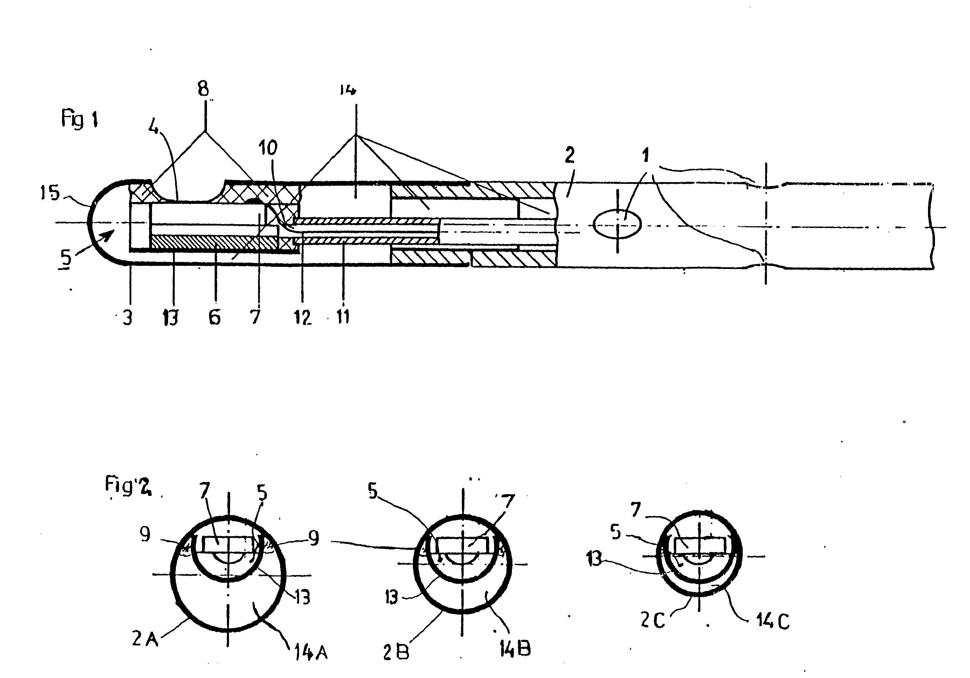

Der Meßfühler 5 hat, wie Fig. 2 zeigt, eine im wesentlichen zylindrische Gestalt und ist auf der der Öffnung 4 abgewandten Seite durch ein gewölbtes Stahlblech 13 abgedeckt. Je nach Durchmesser des Katheters entsteht zwischen dem Meßfühler 5 einerseits und der Innenwand des Katheters ein sichelförmiger Freiraum 14, durch den Flüssigkeiten zugeführt oder entnommen werden oder zusätzliche Leitungen zu weiteren Meßfühlern hindurchgeführt werden können. Ein einheitlicher Meßfühler 5 für unterschiedliche Katheter 2A, 2B und 2C vereinfacht Produktion und Lagerhaltung, zumal der gleiche Fühler sowohl für Messungen am Katheterende (vergl. Fig. 1), als auch für Messungen an anderen Stellen des Katheters verwendet wird. Letzteres ist leicht vorstellbar, wenn man sich den Katheter 2, bezogen auf Fig. 2 links vom Meßfühler 5 nicht durch eine halbkugelförmige Kappe 15 verschlossen, sondern weitergeführt oder zusätzliche Meßfühler 5 hinter den seitlichen Öffnungen 1 angeordnet, vorstellt. Die Katheter 2A, 2B, 2C haben beispielsweise Durchmesser im Verhältnis von 2,67 : 2,33 : 2,0 mm (8F : 7F : 6F), während der Meßfühler 5 einen Durchmesser von 1,67 mm (5F) aufweisen kann oder noch kleiner ist. Der Meßfühler 5 und das Anschlußrohr 11 zusammen werden als Einheit hergestellt und auf Lager gehalten. Je nach Anwendungszweck wird der Meßfühler dann in einen geeigneten Katheter eingeklebt. In Fig. 1 ist das den Meßfühler 5 aufnehmende Stahlrohr 3 auf das Ende des Katheters 2 aufgesetzt und als Abschlußkappe ausgebildet. Stattdessen kann das Rohr 3 als Zwischenstück zwischen zwei Katheterabschnitten eingesetzt sein. Der Meßfühler 5 kann an Stelle des Halbleiter-Druckwandlers 7 oder zusätzlich zu diesem eine oder mehrere andere Fühlerelemente, z. B. einen Sauerstofffühler enthalten, der vorzugsweise vom gleichen Substrat 6 getragen wird.

## Patentansprüche

1. Meßfühler mit einem flachen Halbleiter-Fühlerelement, welcher derart in einen wenigstens eine seitliche Öffnung (4) aufweisenden Katheter (2) eingesetzt ist, daß seine Fühlerfläche der Öffnung gegenübersteht, dadurch gekennzeichnet, daß

a) der Fühler (5) aus einem Träger (6) in Form eines durchgängig längsgeschnittenen Zylinders und aus dem auf der Schnittfläche angeordneten Halbleiter-Fühlerelement (7) besteht ;

b) der Durchmesser des geschnittenen Zylinders (6) kleiner ist als der Innendurchmesser des Katheters (2) ;

c) der Träger (6) an der die Öffnung (4) aufweisenden Seite an der Katheterinnenwand anliegend exzentrisch im Katheter (2) angeordnet und gegenüber dem Katheter abgedichtet ist ; und

d) zwischen dem Fühler (5) und der der Öffnung (4) gegenüberliegenden Innenwand des Katheters (2) ein sich über die gesamte Länge des Fühlers erstreckender, im Querschnitt sichelförmiger Raum (14) gebildet ist.

2. Meßfühler nach Anspruch 1, dadurch gekennzeichnet, daß der Träger (6) auf der der Öffnung (4) abgewandten Seite durch ein kreisförmig gewölbtes Stahlblech (13) abgedeckt ist.

3. Meßfühler nach Anspruch 1 oder 2, dadurch gekennzeichnet, daß die Katheterwand im Bereich des Fühlers (5) durch ein auf das Ende wenigstens eines biegsamen Rohres (2) aufgesetztes, die seitliche Öffnung (4) aufweisendes Metallrohr (3) gebildet ist.

4. Meßfühler nach einem der Ansprüche 1 bis 3, dadurch gekennzeichnet, daß der Meßfühler (5) fest mit einem durch den Katheter (2) hindurchführbaren, elektrische Zuleitungen (12) zum Halbleiter-Fühlerelement (7) umschließenden Anschlußschlauch (11) verbunden ist.

## Claims

1. Measurement sensor with a flat semiconductor sensor element, which is inserted into a catheter (2) having at least one lateral opening (4) such way that its sensing surface faces said opening, characterized in that

a) the sensor (5) consists of a support (6) having the shape of a cylinder cut-through in longitudinal direction, and of the semiconductor sensor element (7) provided on the cut surface ;

b) the diameter of the cut cylinder (6) is smaller than the internal diameter of the catheter (2) ;

c) the support (6) is excentrically provided within the catheter (2) such that it engages that side of the internal wall of the catheter which includes the opening (4) and is sealed against the catheter ;

d) a space (14) sickle-shaped in its cross-section is formed between the sensor (5) and that internal wall of the catheter (2) which is opposite the opening (4), and said space extends along the entire length of the sensor.

2. Measurement sensor according to claim 1, characterized in that the support (6) is covered by a circularily shaped steel sheet (13) on its side which is opposite the opening (4).

3. Measuring sensor according to claim 1 or 2, characterized in that the catheter wall in the region of the sensor (5) is formed by a metal tube (3) comprising the lateral opening (4), with said tube being fixed to the end of a flexible tube (2).

4. Measurement sensor according to one of the claims 1 to 3, characterized in that the measurement sensor (5) is rigidly connected to a flexible connection tube (11) which can be inserted into the catheter (2) and which surrounds the electrical connecting wires (12) of the semiconductor sensor element (7).

## Revendications

1. Capteur de mesure comportant un organe de détection plat à semi-conducteurs, incorporé à un cathéter (2), muni au moins d'une ouverture latérale (4), de manière que sa face de détection se trouve en face de l'ouverture, caractérisé en ce que

a) le capteur (5) comprend un support (6), en forme d'un cylindre coupé sur toute sa longueur et un organe de détection à semi-conducteurs (7) disposé dans le plan de coupe,

b) le diamètre du cylindre coupé (6) est inférieur au diamètre intérieur du cathéter (2),

c) le support (6) est disposé excentriquement dans le cathéter (2), en appui sur sa paroi intérieure du côté muni de l'ouverture (4) et rendu étanche par rapport au cathéter, et

d) un espace (14) dont la section est en forme de croissant et qui s'étend sur toute la longueur du capteur, est ménagé entre le capteur (5) et la paroi intérieure du cathéter (2) opposée à l'ouverture (4).

2. Capteur de mesure selon la revendication 1, caractérisé en ce que le support (6) est recouvert sur le côté opposé à l'ouverture (4) par une tôle d'acier cintrée (13) de forme circulaire.

3. Capteur de mesure selon la revendication 1 ou 2, caractérisé en ce que la paroi du cathéter est constituée dans la zone du capteur (5) par un tube métallique (3) muni de l'ouverture latérale (4), qui est monté au moins à l'extrémité d'un tube flexible (2).

4. Capteur de mesure selon l'une des revendications 1 à 3, caractérisé en ce que le capteur de mesure (5) est rendu solidaire d'un tuyau de raccordement (11) susceptible de traverser le cathéter (2) et entourant des conducteurs électriques (12) menant à l'organe de détection à semi-conducteurs (7).

Fig 1

8    14

4    10    2    1

15

5

3  13  6  7  12  11

Fig 2

7  5          5  7          7
9      9      5
13              13          2C    14C
2A    14A    2B    14B